# EUROPEAN PATENT SPECIFICATION

(11) **EP 1 330 533 B1**
(45) Date of publication and mention of the grant of the patent: **08.02.2006**
(21) Application number: 01972383.2
(22) Date of filing: 03.10.2001
(51) Int. Cl.: C12P 7/02, B01D 15/08

(54) **METHOD OF PREPARING A MODIFIED CORN STEEP LIQUOR PRODUCT**
VERFAHREN ZUR HERSTELLUNG EINES MODIFIZIERTEN MAISQUELLWASSERPRODUKTES
PREPARATION D'UN PRODUIT MODIFIE A PARTIR D'EAU DE MACERATION DU MAIS

(30) Priority: 03.10.2000 ZA 200005371
(43) Date of publication of application: 30.07.2003
(73) Proprietor: SA Bioproducts (PTY) Ltd., 4126 Umbogintwini (ZA); THE BOARD OF SUPERVISORS OF LOUISIANA STATE UNIVERSITY AND AGRICULTURAL AND MECHANICAL COLLEGE, Baton Rouge, LA 70894 (US)
(72) Inventor: VAN WALSEM, Hendrik, Johan, Cambridge, MA 02142-1126 (US); SASKA, Michael, Baton Rouge, LA 70808 (US)
(74) Representative: Curtis, Philip Anthony
(86) International application number: PCT/IB2001/001827
(87) International publication number: WO 2002/029077

(56) References cited:
- EP-A- 0 321 004
- GB-A- 740 205
- US-A- 5 626 847
- PATENT ABSTRACTS OF JAPAN vol. 1998, no. 08, 30 June 1998 (1998-06-30) & JP 10 075795 A (OJI KOONSUTAAC KK), 24 March 1998 (1998-03-24)
- DATABASE WPI Section Ch, Week 199305 Derwent Publications Ltd., London, GB; Class D16, AN 1993-040510 XP002193003 & JP 04 365489 A (MITSUI TOATSU CHEM INC), 17 December 1992 (1992-12-17)
- RANE K D ET AL: "MEMBRANE FILTRATION OF CORN STEEP WATER" CEREAL CHEMISTRY, AMERICAN ASSOCIATION OF CEREAL CHEMISTS. MINNEAPOLIS, US, vol. 78, no. 4, July 2001 (2001-07), pages 400-404, XP001039223 ISSN: 0009-0352

## Description

### BACKGROUND OF THE INVENTION

This invention relates to a method of preparing a modified corn steep liquor product which is enriched in myo-inositol, from a corn steep liquor.

Myo-inositol (also known as inositol, muscle sugar, and hexahydroxycyclohexane) is an important growth factor considered by some as part of the B vitamin group (Szmant, "Industrial utilization of renewable resources", Technomic Publication Company, Lancaster, PA, 1986). It is found in plants both in the free form (in soya and sugar cane for instance) and also as a hexaphosphate, phytin, (in corn for instance) and also as a phosphatidyl derivative, which is a component of vegetable lecithins.

The main applications of myo-inositol are in aquaculture, pet food, animal feed and in the developing markets in health and nutrition.

Corn steep liquor is the traditional industrial source of myo-inositol (e.g Artz and Hach, "Process for refining crude inositol containing solutions and for recovering inositol therefrom", US Patent No 2,615,053, 21 October 1952). In this process, phytin is first precipitated as a calcium salt and then hydrolysed with a strong acid. The production of myo-inositol in this fashion was stopped in the USA in about 1987 because of high production costs. Thus, supplies of myo-inositol now come from China (where it is made from corn steep liquor) and also from Japan (where it is made from rice bran).

US Patent No 3,270,064 to Toyo Koatsu Industries Inc, describes an improvement to the chemical precipitation and hydrolysis process by using a soluble base to raise the pH (e.g ammonia or caustic) and precipitate the phytin. This is followed by hydrolysis under heat and pressure in the presence of an alkaline earth metal carbonate such as magnesium carbonate. Benefits claimed are elimination of complexing reactions and improved purity of the final product. Chemical consumption is still high and the use of caustic or ammonia detracts from the value of the remaining material, as it is not possible to use it as feed.

Because of the chemicals involved in the precipitation and hydrolysis of phytin, any source of the free form of myo-inositol will be at an advantage, provided an efficient way can be found to separate the myo-inositol from the other components. To this effect two processes have been patented, viz. US Patent No 5,096,594 to Rabinowitz and US Patent No 4,482,761 to Chao and Sherman. One process is based on the use of cation exchange resins in Ca²⁺ or Pb²⁺ form with aqueous dimethyl sulfoxide as the mobile phase and the other is based on zeolite molecular sieves. The former process has the disadvantages of using an organic solvent and the use of lead in a food product is entirely unacceptable. The latter process is not very efficient.

JP 100 75 795 is related to method for producing inositol from corn steep water.

Myo-inositol cannot be efficiently separated from monosaccharides (glucose and particularly fructose) on cation exchange resins in monovalent (Na⁺ or K⁺) or divalent (Ca²⁺) form. The use of strong base anion resins to separate myo-inositol from sugar steams has been demonstrated as a feasible option for molasses type streams from the sugar industry (Saska, "Industrial production of inositol: A by-product from the cane molasses desugarization process" 1997, Int. Sugar Jnl., (99): 480-484).

Corn steep liquor cannot be used directly as a feed for sensitive feed applications such as small animals and especially weaning piglets, as the high level of potassium and other salts promote scours and other digestive disorders. Nevertheless, corn steep liquor is a high quality protein source, rich in highly available soluble protein and is of interest in specialist feed applications, provided the problematic components can be separated therefrom.

### SUMMARY OF THE INVENTION

According to the invention there is provided a method of preparing a modified corn steep liquor product from a corn steep liquor which includes suspended solids, high molecular weight compounds, phytic acid, and metal values (Mg, Ca, Fe, Mn, Zn, Na, K), including the steps of:
(a) subjecting the corn steep liquor to ultrafiltration to remove the suspended solids and high molecular weight compounds;
(b) treating the product of step (a) with a phytase enzyme to convert the phytic acid to inositol-mono-phosphate;
(c) treating the product of step (b) with an acid phosphatase enzyme to convert the inositol-mono-phosphate to myo-inositol;
(d) adjusting the pH of the product of step (c) to from 7 to 9 inclusive using a soluble base which causes most or all of the Mg values to precipitate, and removing the precipitate; and
(e) contacting the product of step (d) with a strong acid cation chromatographic resin to produce a first fraction containing most of the remaining metal values, and a second fraction containing most of the myo-inositol, the second fraction being the modified corn steep liquor product.

In step (a), the ultrafiltration is preferably carried out in the range of from 10 000 kD to 100 00 kD inclusive, preferably in the range of from 20 000 kD to 40 000 kD inclusive.

The suspended solids and high molecular weight compounds are removed as they lead to fouling of the resin in step (e).

Any suitable membrane material may be used in the ultrafiltration, for example PVDF (polyvinylidene di-fluoride).

In step (b), the phytase enzyme may be used in free form or may be immobilized onto any suitable carrier.

In step (c) the acid phosphatase enzyme may be used in free form or may be immobilized onto a suitable carrier.

Steps (b) and (c) can be combined into one step, i.e are carried out simultaneously.

In step (d), the pH is preferably adjusted to a value of from 7,5 to 8,5 inclusive.

The preferred base is ammonia as this causes precipitation of struvite which is NH₄MgPO₄.6H₂O, thereby removing substantially all of the Mg values. The Mg values would otherwise interfere with the resin in step (e).

The recovered struvite is a high value slow release fertiliser.

Another base which may be used is potassium hydroxide, which also leads to precipitation of the Mg values present.

In step (e), the first fraction, which may be termed the ash fraction, contains lactate, sulphate, phosphate and chloride values, as well as the remaining metal values.

The ash fraction may also be dried to produce a potassium rich fertilizer. Alternatively, potassium salts may be crystallised from the solution and the remaining solution used as a liquid fertilizer.

The second fraction contains the myo-inositol, as well as other desirable components such as proteins and sugars.

After step (e), the modified corn steep liquor product may be evaporated and dried to produce a dry product, enriched in myo-inositol.

### BRIEF DESCRIPTION OF THE DRAWINGS

- **Figure 1**: is a chromatogram from the Experimental Work; and
- **Figure 2**: is a flow diagram illustrating a continuous simulated moving bed from the Experimental Work.

### DESCRIPTION OF EMBODIMENTS

The crux of the invention is a method of preparing a modified corn steep liquor product from a corn steep liquor which includes suspended solids, high molecular weight compounds, phytic acid and metal values generally in the form of metal ions, the product being enriched in myo-inositol.

The invention will now be described in more detail with reference to the following experimental work:

### Experimental Work

### Step (a) - Ultrafiltration

Crude corn steep liquor (CSL) from a corn wet mill was diluted to 30% (m/m) solids and fed to a Niro ultrafiltration plant with the following operating conditions:
Advanced Membrane Technology Inc. (AMT) spiral wound PVDF membranes of 30 000 kD,
Cross flow velocity 2-4 m/s using 80 mils spacers (2 mm). Pressure drop 1 Bar,
Trans-membrane pressure (TMP) of 4 - 5 Bar,
Concentration ratio (retentate/feed) of 0,5.
Turbidity of permeate (suspended solids) less than 10 NTU (nephelometric turbidity units) as measured by nephelometer.
Feed pH of 4,0,
Temperature of 40°C. -
The result is a product from which suspended solids and high molecular weight compounds, which would lead to fouling of the resin in step (e), are removed.

### Step (b) - Phytase Treatment

The myo-inositol in the clarified CSL from step (a) is essentially present as phytic acid or as a salt thereof. Being a phosphate containing product, this product would follow the ash fraction in any separation, and thus the phytic acid must be converted to free myo-inositol before separation of the ash fraction. This is achieved by enzymatic hydrolysis of the phytic acid to inositol-mono-phosphate using a phytase enzyme. The treatment conditions are as follows:
Enzyme used: BASF Natuphos as described in BASF product brochure in free form,
Enzyme dosage: 500 FTU (phytase activity units) per kg CSL (as 30% solids),
Optimum pH: 3,0 to 4,0 to ensure adequate phytase activity and also shelf life of the CSL,
Temperature of 40°C,
Residence time: 12 hours.

The resulting product contains inositol-mono-phosphate. An analysis of a typical product from step (b) is as follows:

| **Component** | **Units** | **Value** |
|---|---|---|
| **General analyses** | | |
| Density | Kg/m³ | 1150 |
| pH | | 3,0 |
| Dry matter (Brix) | % (m/m) | 31,0 |

| **Organic components** | | |
|---|---|---|
| Glucose | % m/m | 4,21 |
| Frutose | % m/m | 1,68 |
| D-lactate | % m/m | 5,05 |
| L-lactate | % m/m | 5,61 |
| Phytic Acid | % m/m | <1 |
| Inositol-mono-phosphate | % m/m | 5,61 |

| **Amino Acids** | | |
|---|---|---|
| Threonine | % m/m | 0,45 |
| Methionine | % m/m | 0,45 |
| Valine | % m/m | 1,57 |
| Alanine | % m/m | 2,69 |
| Glycine | % m/m | 0,34 |
| Aspartate | % m/m | 0,17 |
| Glutamate | % m/m | 0,59 |
| Serine | % m/m | 0,50 |
| Tyrosine | % m/m | 0,28 |
| Arginine | % m/m | 0,76 |
| Iso-leucine | % m/m | 0,45 |
| Leucine | % m/m | 1,43 |
| Histidine | % m/m | 0,14 |
| Cysteine | % m/m | not measured |
| Phenylalanine | % m/m | 0,84 |

| **Protein composition (dry basis)** | | |
|---|---|---|
| Total free amino acids | % (m/m) | 10,0 |
| TKN protein | % (m/m) | 42,0 |

| **Minerals** | | |
|---|---|---|
| Mg | ppm (m/m) | 14516 |
| Ca | ppm (m/m) | 645 |
| Fe | ppm (m/m) | 210 |
| Mn | ppm (m/m) | 113 |
| Zn | ppm (m/m) | 226 |
| Na | ppm (m/m) | 684 |
| K | % m/m | 6,45 |
| Chlorides | % m/m | 0,98 |
| Free phosphates | % m/m | 1,21 |
| Sulphates | % m/m | 7,43 |

All analyses are expressed on a wet basis unless otherwise stated

### Step (c) - Acid Phosphatase Treatment

An acid phosphatase enzyme is used to hydrolyse the inositol-monophosphate containing a single phosphate group to free myo-inositol and the free phosphate. The acid phosphatase treatment conditions are as follows:
Enzyme used: Sumizyme PM-L from the Shin Nihon Company (activity 3000u/g),
Dosage: 1 unit of activity per g of CSL (as 30% solids),
Temperature of 65°C and pH of 4,0 to 4,5,
Residence time: 24 hours,
Conversion achieved: >98% as determined by inositol and IMP measurement by HPLC.

### Step (d) - pH Adjustment and Removal of Mg Values

It is desirable to conduct the chromatographic resin separation step (Step (e)) without any divalent cations being present. It is well known that these cations have a negative impact on the resolution of the chromatographic separation.

Thus, to avoid this problem, the product of step (c) has its pH adjusted to a value between 7,5 and 8,5 using ammonia, to cause precipitation of struvite.

The precipitate was collected and subjected to an XRF analysis which confirmed the material as being struvite. Analysis of N, Mg and PO₄ using wet chemical assays confirmed the presence of these compounds in the exact stoichiometric quantities to be expected for pure struvite. It was also confirmed that there was very little K (±0,3%) and no Na, Cl, SO₄ or heavy metals present and that the purity was in excess of 99%.

Struvite finds application as a slow release fertiliser.

The clear filtrate was also analysed and it was confirmed that the Mg values were essentially completely removed. This product was then passed on to the next step.

### Step (e) - Chromatographic Separation

The separation was conducted in a single column with the following operating conditions:
Column: 100 cm length with 1 cm diameter,
Resin: Rohm and Haas CR1320 chromatographic resin,
Temperature: 60°C,
Resin volume: 78,5 ml,
Injection volume: 0,01 BV,
Flow rate: 3 ml/min of water (2,25 BV/h or roughly 2,3 m/h velocity),
Detector: On line refractive index (Waters 400).

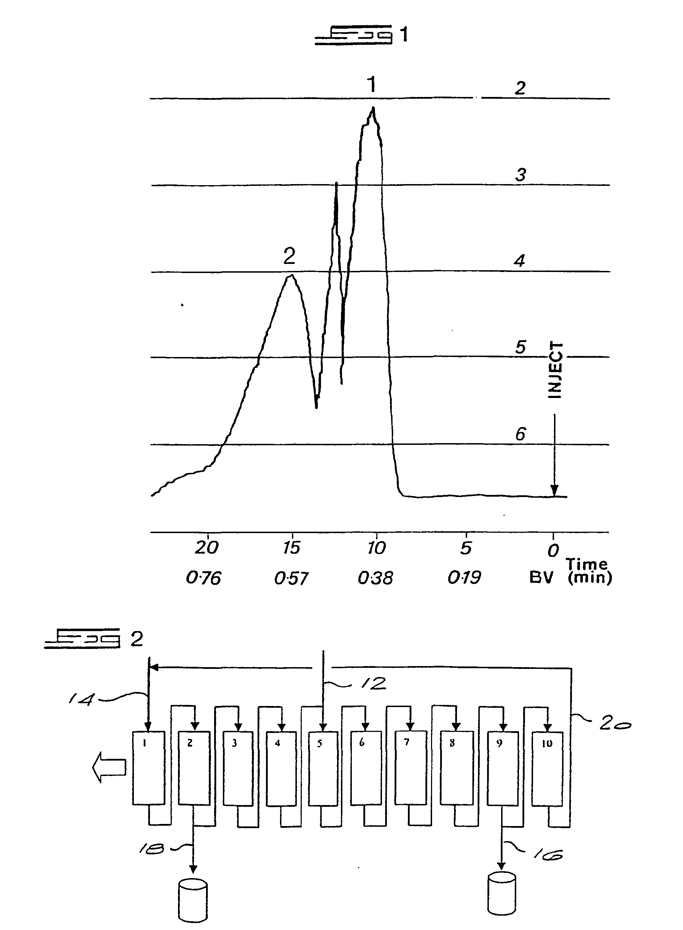

Figure 1 is a chromatogram which shows the separation achieved as follows:

| | |
|---|---|
| Void volume: | 0,32 BV |
| Fraction 1 (raffinate or ash) peak | 0,40 BV |
| Fraction 2 (myo-inositol rich) peak | 0,58 BN |

The peak marked 1 is the ash fraction peak, and the peak marked 2 is the myo-inositol rich fraction peak.

HPLC analyses indicated that the ash fraction contained the lactate, sulphate, phosphate and chloride values and all the cations.

Dry matter determinations indicated that the total solids of the CSL was split equally between fractions 1 and 2, with all the myo-inositol reporting to fraction 2. The concentration of myo-inositol was 10% on a dry matter basis.

In order to optimise the chromatographic separation it is desirable to operate a continuous device such as a simulated moving bed (SMB). In this way it is possible to optimise the eluent and feed flows to achieve the best separation with minimum dilution, whilst maintaining steady operation by effectively maintaining the separation peaks in a stationary position. This means that the two fractions can be extracted from fixed ports as is illustrated in Figure 2 which is described in more detail below.
The design flowrates as indicated were determined from the batch chromatogram (pulse test) discussed above, using standard design and scale-up calculations as used by Advanced Separation Technologies (a division of Calgon Carbon) to design separations based on their Isep rotating carousel system. The design criteria are as follows:

| | |
|---|---|
| Resin volume: | 2,2 litres in total (220 ml per column) |
| Step time: | 4min |
| Resin flow: | 55 ml/min |
| Linear velocity: | 7,5 m/h (safe value for resin of 320 micron) |
| Bed depth: | 0,45 m |
| Feed rate: | 0,1 BV/h |
| Slow Product: | Fraction 2 of Figure 2 being the myo-inositol enriched fraction |
| Fast Product: | Fraction 1 of Figure 2 being the ash containing fraction. |

Referring to Figure 2, there is shown a pilot set-up for continuous separation of myo-inositol enriched CSL.

The product of step (d) is fed into port 5 of the apparatus via a line 12, at a feed rate of 4 ml/min. Water is fed into port 1 of the apparatus via a line 12, at a feed rate of 27,5 ml/min.

Fraction 1 is removed from the apparatus at port 9 via a line 16, at a rate of 9,35 ml/min.

Fraction 2 is removed from the apparatus at port 2 via a line 18 at a rate of 22,15 ml/min.

Product from port 10 is recycled via a line 20 to the water inlet line 14.
The numbers 1 to 10 in the drawing refer to stationary ports. Each of the ten columns containing the resin in the apparatus moves from port to port with a step time of 4 minutes. At each step, each column moves to the next port, and in this way continuous countercurrent resin flow is achieved. The resin flow is 220 ml resin/4 min, or 55 ml/min.

### Crystallisation of Potassium Salts from the First Fraction

A small sub-sample of the concentrated first fraction was further concentrated under vacuum in a rotary laboratory evaporator in order to investigate the feasibility of crystallizing potassium salts. Fine needle like crystals of some 50 x 300 microns in size were produced and a few grams recovered with a laboratory centrifuge. The crystals were determined by powder X-ray diffraction analysis to be primarily a potassium hydrogen phosphate KH₂PO₄ (arcanite), with a smaller amount of potassium sulfate K₂SO₄.

## Claims

1. A method of preparing a modified corn steep liquor product from a corn steep liquor which includes suspended solids, high molecular weight compounds, phytic acid, and metal values including the steps of:
(a) subjecting the corn steep liquor to ultrafiltration to remove the suspended solids and high molecular weight compounds;
(b) treating the product of step (a) with a phytase enzyme to convert the phytic acid to inositol-mono-phosphate;
(c) treating the product of step (b) with an acid phosphatase enzyme to convert the inositol-mono-phosphate to myo-inositol;
(d) adjusting the pH of the product of step (c) to from 7 to 9 inclusive using a soluble base which causes most or all of the Mg values to precipitate, and removing the precipitate; and
(e) contacting the product of step (d) with a strong acid cation chromatographic resin to produce a first fraction containing most of the remaining metal values, and a second fraction containing most of the myo-inositol, the second fraction being the modified corn steep liquor product.

2. A method according to claim 1 wherein in step (a) the ultrafiltration is carried out in the range of from 10 000 kD to 100 000 kD inclusive.

3. A method according to claim 2 wherein in step (a) the ultrafiltration is carried out in the range of from 20 000 kD to 40 000 kD inclusive.

4. A method according to any one of claims 1 to 3 wherein in step (a) the ultrafiltration is carried out using a polyvinylidene di-fluoride membrane material.

5. A method according to any one of claims 1 to 4 wherein in step (b) the phytase enzyme is used in free form or immobilized onto a carrier.

6. A method according to any one of claims 1 to 5 wherein in step (c) the acid phosphatase enzyme is used in free form or immobilized on a carrier.

7. A method according to any one of claims 1 to 6 wherein steps (b) and (c) are carried out simultaneously.

8. A method according to any one of claims 1 to 7 wherein in step (d) the pH is adjusted to a value of from 7.5 to 8.5 inclusive.

9. A method according to any one of claims 1 to 8 wherein in step (d) the soluble base is ammonia.

10. A method according to any one of claims 1 to 8 wherein in step (d) the soluble base is potassium hydroxide.

11. A method according to any one of claims 1 to 10 wherein after step (e) the modified corn steep liquor product is evaporated and dried to produce a dry produce enriched in myo-inositol.

12. A method according to any one of claims 1 to 11 wherein after step (e) potassium salts are crystallised from the first fraction.

## Patentansprüche

1. Verfahren zum Herstellen eines modifizierten Getreidequellwasserproduktes aus einem Getreidequellwasser, das suspendierte Feststoffe, Verbindungen mit hohem Molekulargewicht, Phytinsäure und metallische Inhaltsstoffe aufweist, aufweisend die Schritte:
(a) Unterwerfen des Getreidequellwassers der Ultrafiltration, um die suspendierten Feststoffe und die Verbindungen mit hohem Molekulargewicht zu entfernen,
(b) Behandeln des Produktes von Schritt (a) mit einem Phytaseenzym, um die Phytinsäure in Inositmonophosphat umzuwandeln,
(c) Behandeln des Produktes von Schritt (b) mit einem sauren Phosphataseenzym, um das Inositmonophosphat in myo-Inosit umzuwandeln,
(d) Einstellen des pH-Wertes des Produktes von Schritt (c) auf 7 bis einschließlich 9 unter Benutzung einer löslichen Base, die bewirkt, daß die meisten oder alle Mg-Inhaltsstoffe ausgefällt werden, und Entfernen des Niederschlags, und
(e) Inberührungbringen des Produktes von Schritt (d) mit einem stark sauren chromatographischen Kationenharz, um eine erste Fraktion zu erzeugen, die das meiste der verbleibenden metallischen Inhaltsstoffe enthält, und eine zweite Fraktion, die das meiste des myo-Inosits enthält, wobei die zweite Fraktion, das modifizierte Getreidequellwasserprodukt ist.

2. Verfahren nach Anspruch 1, wobei in Schritt (a) die Ultrafiltration in dem Bereich von 10.000 kD bis einschließlich 100.000 kD durchgeführt wird.

3. Verfahren nach Anspruch 2, wobei in Schritt (a) die Ultrafiltration in dem Bereich von 20.000 kD bis einschließlich 40.000 kD durchgeführt wird.

4. Verfahren nach einem der Ansprüche 1 bis 3, wobei in Schritt (a) die Ultrafiltration unter Benutzung eines Polyvinylidendifluorid-Membranmaterials durchgeführt wird.

5. Verfahren nach einem der Ansprüche 1 bis 4, wobei in Schritt (b) das Phytaseenzym in freier Form oder auf einem Träger immobilisiert benutzt wird.

6. Verfahren nach einem der Ansprüche 1 bis 5, wobei in Schritt (c) das saure Phosphataseenzym in freier Form oder auf einem Träger immobilisiert benutzt wird.

7. Verfahren nach einem der Ansprüche 1 bis 6, wobei Schritt (b) und (c) gleichzeitig durchgeführt werden.

8. Verfahren nach einem der Ansprüche 1 bis 7, wobei in Schritt (d) der pH-Wert auf einen Wert von 7,5 bis einschließlich 8,5 eingestellt wird.

9. Verfahren nach einem der Ansprüche 1 bis 8, wobei in Schritt (d) die lösliche Base Ammoniak ist.

10. Verfahren nach einem der Ansprüche 1 bis 8, wobei in Schritt (d) die lösliche Base Kaliumhydroxid ist.

11. Verfahren nach einem der Ansprüche 1 bis 10, wobei nach Schritt (e) das modifizierte Getreidequellwasserprodukt eingedampft und getrocknet wird, um ein trockenes, mit myo-Inosit angereichertes Produkt zu herzustellen.

12. Verfahren nach einem der Ansprüche 1 bis 11, wobei nach Schritt (e) aus der ersten Fraktion Kaliumsalze auskristallisiert werden.

## Revendications

1. Procédé de préparation d'un produit d'eau de trempage de maïs modifié à partir d'une eau de trempage de maïs qui comprend des matières solides en suspension, des composés ayant un poids moléculaire élevé, de l'acide phytique et des valeurs métalliques comprenant les étapes suivantes :
(a) soumettre l'eau de trempage de maïs à une ultrafiltration pour retirer les matières solides en suspension et les composant de poids moléculaire élevé ;
(b) traiter le produit de l'étape (a) avec une enzyme phytase pour transformer l'acide phytique en monophosphate d'inositol ;
(c) traiter le produit de l'étape (b) avec une enzyme phosphatase acide pour transformer le monophosphate d'inositol en myo-inositol ;
(d) ajuster le pH du produit de l'étape (c) de 7 à 9 inclus en utilisant une base soluble qui engendre la précipitation de la plupart ou de toutes les valeurs Mg, et retirer le précipité ; et
(e) mettre en contact le produit de l'étape (d) avec une forte résine acide chromatographique à cations pour produire une première fraction contenant la plupart des valeurs métalliques restantes, et une seconde fraction contenant la plupart du myo-inositol, la seconde fraction étant le produit d'eau de trempage de maïs modifié.

2. Procédé selon la revendication 1, dans lequel à l'étape (a), l'ultrafiltration est réalisée dans la fourchette de 10 000 kD à 100 000 kD inclus.

3. Procédé selon la revendication 2, dans lequel à l'étape (a), l'ultrafiltration est réalisée dans la fourchette de 20 000 kD à 40 000 kD inclus.

4. Procédé selon l'une quelconque des revendications 1 à 3, dans lequel à l'étape (a), l'ultrafiltration est réalisée en utilisant un matériau à membrane de di-fluorure de polyvinylidène.

5. Procédé selon l'une quelconque des revendications 1 à 4, dans lequel à l'étape (b), l'enzyme phytase est utilisée selon une forme libre ou immobilisée sur un support.

6. Procédé selon l'une quelconque des revendications 1 à 5, dans lequel à l'étape (c), l'enzyme phosphatase acide est utilisée selon une forme libre ou immobilisée sur un support.

7. Procédé selon l'une quelconque des revendications 1 à 6, dans lequel les étapes (b) et (c) sont réalisées simultanément.

8. Procédé selon l'une quelconque des revendications 1 à 7, dans lequel à l'étape (d) le pH est ajusté à une valeur de 7,5 à 8,5 inclus.

9. Procédé selon l'une quelconque des revendications 1 à 8, dans lequel à l'étape (d), la base soluble est de l'ammoniac.

10. Procédé selon l'une quelconque des revendications 1 à 8, dans lequel à l'étape (d) la base soluble est de l'hydroxyde de potassium.

11. Procédé selon l'une quelconque des revendications 1 à 10, dans lequel après l'étape (e), le produit d'eau de trempage de maïs modifié est évaporé et séché de manière à produire un produit sec enrichi en myo-inositol.

12. Procédé selon l'une quelconque des revendications 1 à 11, dans lequel après l'étape (e), des sels de potassium sont cristallisés à partir de la première fraction.
